Europäisches Patentamt

European Patent Office

Office européen des brevets

(19)

(11)  **EP 1 157 672 A1**

(12)  # EUROPEAN PATENT APPLICATION

(43) Date of publication:
  **28.11.2001   Bulletin 2001/48**

(51) Int Cl.[7]: **A61F 2/00**, A61L 9/01,
  A61L 15/46, A23L 3/3427,
  B65D 81/26

(21) Application number: **00110884.4**

(22) Date of filing: **23.05.2000**

(84) Designated Contracting States:
  **AT BE CH CY DE DK ES FI FR GB GR IE IT LI LU
  MC NL PT SE**
  Designated Extension States:
  **AL LT LV MK RO SI**

(71) Applicant: **THE PROCTER & GAMBLE COMPANY
  Cincinnati, Ohio 45202 (US)**

(72) Inventors:
  • **Carlucci, Giovanni
    66100 Chieti (IT)**
  • **Fabrizio, Giuliana
    65010 Carpineto Della Nora (Pescara) (IT)**

  • **Gagliardi, Ivano
    65123 Pescara (IT)**
  • **Gonzales, Denis Alfred
    65123 Pescara (IT)**
  • **Klabbers, Victor
    1850 Grimbergen (BE)**
  • **S'heeren, Gert Erik Erwin
    3980 Tessenderlo (BE)**

(74) Representative:
  **Kremer, Véronique Marie Joséphine et al
  Procter & Gamble
  European Service GmbH
  Sulzbacher Strasse 40
  65824 Schwalbach am Taunus (DE)**

(54)  **Liquid and odour absorbent structure for inanimate places such as refrigerators**

(57)    A method of containment of odor and liquid in an inanimate place is disclosed by disposing in said place, a disposable absorbent structure in the form of sheet, this absorbent structure comprising an odor control means and having a water absorption capacity of at least 0.06 grams per square cm. This method is particularly suitable for kitchen applications like the control of liquid and odor in refrigerators. Suitable layered absorbent structures for use in inanimate places are also disclosed.

Fig.3

**Description**

Field of the Invention

This invention relates to a method of containment of odour and liquid in an inanimate place, by disposing in said place a layered absorbent structure able to neutralise odour and absorb and retain liquid as well as to layered absorbent structures suitable for such an application.

Background of the Invention

Inanimate places can be contaminated by unpleasant odor and spillage. This typically occurs when food are manipulated and prepared (e.g., in the kitchen), stored (e.g., in refrigerators, cupboards, vegetable and fruits baskets, picnic baskets, lunch boxes) or transported (e.g., in cars from shopping center to the consumer home), or when food wastes are disposed off (e.g., in paper or plastic trash bags). The need of controlling not only odor but also liquid also arises when storing soiled laundry, garments and especially sportswear and footwear after use. Indeed the storage of wet laundry (e.g., footwear, washcloth, facecloth, sportswear) typically in laundry bags facilitates the development of strong and unpleasant odors. The need of controlling odor and liquid also arises in toilets environment which might be contaminated by urine spillage.

Given the foregoing there is a need for an economical and easy means for containing both odor and fluid in inanimate places.

Odor absorbing systems have been developed and are presently sold on the marketplace. Such systems are for instance available in various forms like in the form of perforated plastic containers containing an activated carbon-containing paper bag or in the form of paper sheet like products. For instance US5782409, US 5611486 or US5046604 disclose sheet materials with odor absorbing means like activated carbon, and/or zeolite and/or silica and the like. However none of these patent references disclose odor controlling sheet like products being effectively suitable for absorbing and retaining liquid. Actually none of these references recognizes the dual problem of both containing odor and liquid in inanimate places like for example kitchens and especially refrigerators, nor addresses such a problem.

Co-pending patent application number PCT/US99/26969 discloses containers for use in the disposal of food wastes, these containers are composed of liquid impervious walls which have inner and outer surfaces and having placed inside the containers an absorbent material. Such a container preferably also comprises a means for closing and sealing itself. The absorbent material has deposited thereon an effective amount of an odor-neutralizing composition.

It is an object of the present invention to provide a means to absorb both odor and liquid and hence control odor and liquid in an inanimate place accidentally contaminated by unpleasant odor and spillage like for instance refrigerators contaminated by aging food like vegetable or fruit or toilets surroundings contaminated by urine spillage. It is a further object of the present invention to provide such a means in an economical and easy way.

It has now been found that this can be achieved by disposing in an inanimate place a disposable absorbent structure in the form of a sheet, this absorbent structure comprising an odor control means and having a water absorption capacity of at least 0.06 grams per cm$^2$. Typically this absorbent structure is a layered absorbent structure comprising at least one layer of fibrous material as a substrate for the odor control means. More particularly, the layered absorbent structure comprises a first and a second layer of fibrous material and an intermediate layer between the first and second layer of fibrous material, the intermediate layer comprising an odor control means, this absorbent structure having a water absorption capacity of at least 0.06 grams per cm$^2$.

In a preferred embodiment the absorbent structure suitable for use according to the present invention has a water absorption capacity of at least 2 grams per gram of the absorbent structure. Particularly suitable absorbent structures for use herein comprise absorbing gelling material beside the odor control means. The presence of absorbent gelling materials increases the absorption and retention capacity of the absorbent structure.

Advantageously the use of the absorbent structures according to the present invention results in a more easy cleaning of inanimate places like hard-surfaces found typically in house, e.g., in kitchens and especially refrigerators. By avoiding the soiling otherwise associated to spillage that might occur with for example aging food like vegetables or fruits, the cleaning operation is facilitated and/or postponed, i.e., for example refrigerators will need to be cleaned less often in presence of such absorbent structures.

Advantageously the use of the absorbent structures according to the present invention on inanimate places results in reduced development of microorganisms like bacteria and/or fungi on such inanimate places. Indeed it is speculated without being bound by any theory, that the absorption of liquid and malodorous components, reduces the availability of nutriments (e.g., water) otherwise necessary for microbial growth, thereby reducing microbial contamination on such inanimate places (e.g., kitchen surfaces, refrigerators and the like).

In a preferred embodiment the absorbent structures according to the present invention are breathable, i.e.,

are air and vapor permeable. It has been observed that a breathable environment further helps the odor control capacity of the structures. Such breathable absorbent structures are particularly preferred as odors carr effectively be absorbed from both outer sides of the structures, e.g., from the topsheet side of the first fibrous layer (which is the side of the first fibrous layer facing away from the intermediate layer) and from the backsheet side of the second fibrous layer of the absorbent structures (which is the side of the second fibrous layer facing away from the intermediate layer). A particular suitable application of such a breathable absorbent structure is when it is disposed in refrigerators especially on refrigerators grilles or for example in the middle of a laundry bag containing soiled linen.

The absorbent structures according to the present invention may comprise additional layers beside the layer of fibrous material (typically the first and second fibrous layers), including topsheet and backsheet. Such topsheet and backsheet may comprise a single layer or a multiplicity of layers. Such topsheet and backsheet might be chosen to meet any desired function, they might be air and/or water vapor permeable and/or liquid impermeable and the like. In preferred execution herein the topsheet and backsheet are air and water vapor permeable to not impair on the breathability of the whole structure, when such function is desired. In an embodiment of the present invention the backsheet is liquid impermeable to prevent that liquid that has been absorbed and is contained in the absorbent structures, leaks through the structures and/or wets the user hand upon use of such structures as cleaning sheet/wipe for inanimate surfaces like kitchen surfaces. Preferably the absorbent structures herein might advantageously been provided in the form of soft structures which are readily flexible and have a pliable drapeable hand such that they can easily be used to clean spills.

The absorbent structures according to the present invention might be provided with antislipping properties on the outer side of a fibrous layer or on the outer side of any additional layers, topsheet and/or backsheet, in the execution herein wherein such additional layers are present. By 'outer side' it is meant the side facing away from the intermediate layer. Indeed the outer side of a fibrous layer or the outer side of the topsheet or backsheet may have a coefficient of friction of at least 0.5 towards metal and/or 0.6 towards cotton when measured according to standard method ASTM D1894. This advantageously provides stay in place properties to the absorbent structures allowing various usage possibilities. Alternatively the absorbent structures might be provided with attachment means in the form of conventional auto-adhesive or pressure sensitive-adhesive. This advantageously would allow the use of the absorbent structures according to the present invention even in non-horizontal applications such as vertical or inverted surfaces in e.g., cupboards, waste bins and the like.

The absorbent structures according to the present invention might comprise an indicator suitable to indicate to the user when the structures have to be replaced by new ones for effective odor and liquid containment, i.e., indicates the end of the life time of the absorbent structures. The indicator might be triggered by time of exposure to the contaminated inanimate places or more preferably it might be triggered by loading/saturation of the water absorption capacity and/or odor control capacity of the absorbent structures. Any conventional means known to those skilled in the art and compatible with the absorbent structures according to the present invention might be suitable to use for such function.

The absorbent structures according to the present invention might be able to deliver fragrance or perfume on top of their ability to control both odor and liquid. Indeed a 'scent signal' in the form of a pleasant odor which signals the removal of odor during use of the absorbent structure might be present. Any conventional means known to those skilled in the art and compatible with the absorbent structures according to the present invention might be suitable to use for such function.

In a preferred execution of the present invention the absorbent structures are able to be used for multiple usage conditions where various sizes of the structures might be desirable. Advantageously the absorbent structure according to the present invention is easily sizable on demand depending on user needs (e.g., for small places applications like shoes or big places applications like refrigerators shelves or as floor carpet), this without occurrence of any spill of the actives material like odor control particles and/or absorbent gelling material particles. The versatility in sizing of the absorbent structure is achieved by separating the areas formed by the discontinuous intermediate layer by so called bonding areas. These bonding areas might be provided with any separation means indicating to the user where to cut/size the absorbent structure. This can be in the form of visually recognizable lines/signs to be cut by the user with the help of scissors or by means allowing cuttability by applying simply forces thereto, like tear-off systems, typically pre-perforated lines.

Brief description of the drawings

The invention is further described with reference to the accompanying drawings:

Figure 1 is a perspective view of a layered absorbent structure suitable to be used in the method of containment of liquid and odor according to the present invention, with the first layer of fibrous material partially raised.

Figure 2 is a sectional view of the absorbent structure taken on the line I-I of Figure 1.

Figure 3 is a perspective view of a layered absorbent structure according to the present invention with discontinuous areas comprising the intermediate layer, said areas being separated by bonding areas where the first and the second layers of fibrous material are bonded together, the first layer of fibrous material is partially raised.

Figure 4 is a sectional view of the absorbent structure taken on the line II-II of Figure 3.

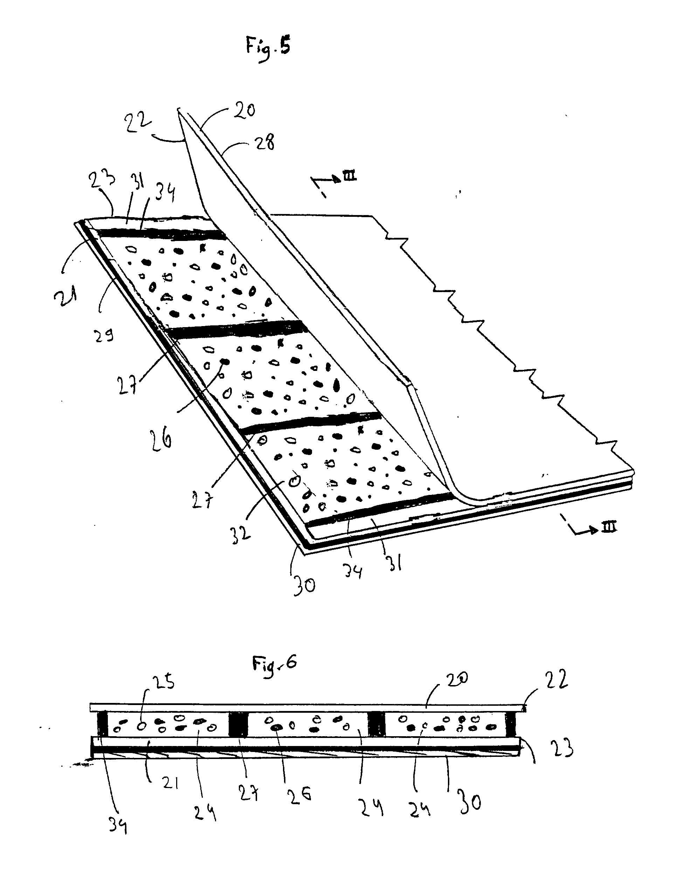

Figure 5 is a perspective view of a layered absorbent structure according to the present invention comprising first and second layer of fibrous material and a discontinuous intermediate layer between the first and second layer, the discontinuous intermediate layer forms areas being separated by bonding areas where the first and the second layer of fibrous material are bonded together, the first layer of fibrous material is partially raised. The absorbent structure comprising a backsheet overlaying the second layer of fibrous material on the side facing away from the discontinuous intermediate layer.

Figure 6 is a sectional view of the absorbent structure taken on the line III-III of Figure 5.

<u>Summary of the Invention</u>

In its broadest aspect the present invention relates to a method of containment of odor and fluid in an inanimate place by disposing in said place a disposable absorbent structure in the form of a sheet, this absorbent structure comprising an odor control means and having a water absorption capacity of at least 0.06 grams per $cm^2$.

The present invention further encompasses a layered absorbent structure, particularly suitable for neutralizing odor and absorbing liquid in inanimate places, comprising a first and a second layer of fibrous material and a discontinuous intermediate layer between said first and second layer of fibrous material, the discontinuous intermediate layer comprising carbon material and a water absorbing means, the discontinuous intermediate layer defining areas which are separated by bonding areas where the first and second layer of fibrous material are bonded together.

The present invention also encompasses a layered absorbent structure, particularly suitable for neutralizing odor and absorbing liquid in inanimate places, comprising a first and a second layer of fibrous material and a discontinuous intermediate layer between said first and second layer of fibrous material, the discontinuous intermediate layer comprising an odor control means and a water absorbing means, the discontinuous intermediate layer defining areas separated by bonding areas where the first and second layer of fibrous material are bonded together, the absorbent structure further comprising a backsheet overlaying the second layer of fibrous material on the side facing away from said intermediate layer.

The present invention further encompasses a layered absorbent structure, particularly suitable for neutralizing odor and absorbing liquid in inanimate places, comprising a first and a second layer of fibrous material and a discontinuous intermediate layer between said first and second layer of fibrous material, the discontinuous intermediate layer comprising an odor control means and a water absorbing means, the discontinuous intermediate layer defining areas separated by bonding areas where the first and second layer of fibrous material are bonded together, the absorbent structure further comprising a means to provide a 'scent signal' in the form of a pleasant odor which signals the removal of odor during use of the absorbent structure.

The present invention further encompasses a layered absorbent structure, particularly suitable for neutralizing odor and absorbing liquid in inanimate places, comprising a first and a second layer of fibrous material and a discontinuous intermediate layer between said first and second layer of fibrous material, the discontinuous intermediate layer comprising an odor control means and a water absorbing means, the discontinuous intermediate layer defining areas separated by bonding areas where the first and second layer of fibrous material are bonded together, the absorbent structure further comprising attachment means.

The present invention further encompasses an absorbent structure, particularly suitable for neutralizing odor and absorbing liquid in inanimate places, said absorbent structure comprising an odor control means and having a water absorption capacity of at least 0.06 grams per square cm, the absorbent structure being provided with antislipping properties.

Finally the present invention also encompasses an absorbent structure, particularly suitable for neutralizing odor and absorbing liquid in inanimate places, said absorbent structure comprising an odor control means and having a water absorption capacity of at least 0.06 grams per square cm, the absorbent structure being provided with an indicator which indicates the end of the life time of the absorbent structure.

Detailed description of the Invention

By "containment of odour" it is meant herein control and/or neutralisation of odour. Malodour detection can be reduced or even prevented by various mechanisms depending on the odour control means used. Any means known to those skilled in the art to control odour are suitable for use herein as described in more details herein after.

By "containment of liquid" it is meant herein absorption and retention of liquid.

"Inanimate places" is used herein in contrast to animate places, i.e., those being in direct contact to human body. Inanimate places include, but are not limited to, any household application, i.e. include any place in the house like kitchens, bathrooms, toilets, cupboards, wardrobe, waste bins, ashtray, as well as appliances like refrigerators, microwave oven. "Inanimate places" also include any application outside the house, like cars, picnic baskets, lunch baskets, sport and travel bags. "Inanimate places" also include various other applications like pet area, laundry bag, shoes especially sportshoes after use.

The term "disposable" is used herein to describe structures which are not intended to be launched or otherwise restored or reused as an absorbent structure (i.e., they are intended to be discarded after a single use and, preferably to be recycled, composted or otherwise disposed of in an environmentally compatible manner, typically they are disposed in waste bins where they might still provide some odour and liquid containment properties).

The present invention is based on the finding that disposing a disposable sheet-like absorbent structure comprising an odour control means and having a water absorption capacity of at least 0.06 grams per $cm^2$, in an inanimate place contaminated by odour and spillage, effectively contains odour and liquid.

The absorbent structures according to the present invention are particularly suitable to be disposed in refrigerators serving as a disposable refrigerator refreshing sheet, or more generally on any hard surface serving as a protection sheet against contamination of odour and spillage and hence against soiling. A particular application of such a protection sheet also includes the surroundings of toilets (e.g., walls and/or floors) serving as disposable floor/wall carpets.

The fibrous layers suitable for use in the absorbent structures according to the present invention might be any fibrous material known to those skilled in the art. Suitable fibrous materials for use herein include natural, modified or synthetic fibers, particularly modified or non-modified cellulose fibers, in the form of fluff and/or tissues. Modified cellulose fibers such as the stiffened cellulose fibers can be used. Synthetic fibers suitable for use herein include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Typically the fibrous materials are one or more layers of wadding, absorbent paper or woven or nonwoven layers. These fibrous materials might contain a more or less high numbers of apertures. The apertures in said layer may be of any configuration and may also be of varying dimensions. The apertures preferably are evenly distributed across the entire surface of the layer, however layers having only certain regions of the surface having apertures are also envisioned. Preferably, the fibrous layer surfaces are hydrophilic or are treated to be hydrophilic. The fibrous layers are preferably made of dry-formed layers, generally referred to as 'air laid' layers, of short cellulose fibres having a basic weight of between 20g/m2 and 150g/m2, preferably between 40g/m2 and 120g/m2, and more preferably between 50g/m2 and 70g/m2. Alternatively the fibrous layers may consist of a dry-formed mixture of cellulose fibres and monocomponent fibres, bicomponent fibres or tricomponent fibres. Example of dry-formed mixture of cellulose and bicomponent polyethylene/polypropylene fibres are those sold by Danaklon a/s of Varde, Denmark as AL Thermal E® and AL-Thermal C®.

The presence of an odour control means is an essential feature of the absorbent structures according to the present invention. Any means able to control odour are suitable for use herein especially any odour control agent.

Typically, the absorbent structures according to the present invention comprise the odor control agent or a mixture thereof at a level of from 10 gm-2 to 600 gm-2, preferably from 50 to 300 gm-2, more preferably from 100 gm-2 to 200 gm-2 and most preferably from 120 gm-2 to 150gm-2

Odour control means for use herein include any odour control agent or combinations thereof, known in the art for this purpose. These agents can typically be classified with respect to the mechanism by which the malodor detection is reduced or prevented. For example, odor control agents which chemically react with malodorous compounds or with compounds which produce malodorous degradation products thereby generating compounds lacking odor or having an odor acceptable to consumers may also be utilized herein. Odor control agents which mask malodors or odor control agents having a certain structural configuration that enables them to absorb and thus eliminate a broad array of odoriferous molecules as well as those which prevent the formation of odoriferous molecules like antimicrobial agents might be used herein too.

More generally the odor control agents might be classified as being "direct " odor control agents or "indirect" odor control agents. Direct odor control agents are molecules possessing the ability to neutralize odoriferous molecules. Indirect odor control agents are those components which prevent the formation of odoriferous molecules for example by interrupting the biological processes responsible for the formation of such odoriferous molecules. Such indirect odor control agents do not have the ability of reducing malodors that have already been produced.

Highly preferred odor control agents for use herein are odor absorbing agents, i.e. agents that control odoriferous molecules by absorbing them. Suitable odor absorbing agents for use herein typically include, carbon materials, baking soda, natural and synthetic clays, kieselguhr, zeolites, silicas, bentonites, starch, diatomaceous earth, cyclodextrine and derivatives thereof. Cyclodextrin and derivatives thereof is for example more fully described in US 5593670. Alternative odor control absorbent agents include mucopolysaccharides like chitin and chitosan and derivatives thereof including modified- chitin or chitosan, salts of chitin or chitosan, crosslinked chitosan or chitin. Ion exchange resins such as those described in US 4 289 513 and US 3340875 might also be used herein.

Suitable odor control agents also include chelating agents typically suitable to chelate metal ions like iron and manganese. Such chelating agents can be selected from the group consisting of phosphonate chelating agents, polyfunctionally-substituted aromatic chelating agents, amino carboxylate chelating agents, other chelating agents like ethylene diamine N,N'- disuccinic acid, aspartic acid, glutamic acid, malonic acid, glycine and mixtures thereof. Suitable phosphonate chelating agents to be used herein may include ethydronic acid, alkali metal ethane 1-hydroxy diphosphonates as well as amino phosphonate compounds, including amino alkylene poly (alkylene phosphonate), alkali metal ethane 1-hydroxy diphosphonates, nitrilo trimethylene phosphonates, ethylene diamine tetra methylene phosphonates, aminotri(methylene phosphonates) (ATMP) and diethylene triamine penta methylene phosphonates. The phosphonate compounds may be present either in their acid form or as salts of different cations on some or all of their acid functionalities. Preferred phosphonate chelating agents to be used herein are diethylene triamine penta methylene phosphonates (DETPMP). Such phosphonate chelating agents are commercially available from Monsanto under the trade name DEQUEST® . Polyfunctionally-substituted aromatic chelating agents may also be useful herein. See U.S. patent 3,812,044, issued May 21, 1974, to Connor et al. Preferred compounds of this type in acid form are dihydroxydisulfobenzenes such as 1,2-dihydroxy -3,5-disulfobenzene. A preferred biodegradable chelating agent for use herein is ethylene diamine N,N'- disuccinic acid, or alkali metal, or alkaline earth, ammonium or substitutes ammonium salts thereof or mixtures thereof. Ethylenediamine N,N'- disuccinic acids, especially the (S,S) isomer have been extensively described in US patent 4, 704, 233, November 3, 1987. to Hartman and Perkins. Ethylenediamine N,N'- disuccinic acids is, for instance, commercially available under the tradename ssEDDS® from Palmer Research Laboratories. Suitable amino carboxylate chelating agents to be used herein include ethylene diamine tetra acetates (EDTA), diethylene triamine pentaacetates, diethylene triamine pentaacetate (DTPA), N-hydroxyethylethylenediamine triacetates, nitrilotri-acetates, ethylenediamine tetrapropionates, triethylenetetraaminehexa-acetates, diethylenetriamine pentaacetates, ethanol-diglycines, propylene diamine tetracetic acid (PDTA) and methyl glycine di-acetic acid (MGDA), both in their acid form, or in their alkali metal, ammonium, and substituted ammonium salt forms. Particularly suitable amino carboxylates to be used herein are ethylene diamine tetra acetates (EDTA), diethylene triamine penta acetic acid (DTPA) or mixture thereof.

Other odor control agents also include carbonates (e.g., sodium carbonate), bicarbonates (e.g., sodium bicarbonate), phosphates (e.g., sodium phosphate), sulphates (e.g., zinc and copper sulphates), carboxylic acids such as citric acid, lauric acid, boric acid, adipic acid and maleic acid and the like. PH buffering means might also be used herein. PH buffering means for use herein include citric acid and sodium bicarbonate or sodium phosphate and sorbic acid. Masking agents such as perfumes may also be used as odor control agents herein.

Antimicrobial agents might also be used herein as the odor control agent including biocidal compounds, i.e., substance that kill microorganisms or biostatic compounds, i.e., substance that inhibit and/or regulate the growth of microorganisms.

Highly preferred odor control agents for use herein are carbon material, silicate material and zeolite material either alone or in mixture.

The use and manufacture of zeolite material is well know in the literature and is described in the following reference texts: ZEOLITE SYNTHESIS, ACS Symposium Series 398, Eds. M. L. Occelli and H. E Robson (1989) pages 2-7; ZEOLITE MOLECULAR SIEVES, Structure, Chemistry and Use, by D. W. Breck, John Wiley and Sons (1974) pages 245-250, 313-314 and 348-352; MODERN APPLICATIONS OF MOLECULAR SIEVE ZEOLITES, Ph.D. Dissertation of S. M. Kuznicki, U. of Utah (1980), available from University of Microfilms International, Ann Arbor, Michigan, pages 2-8. Zeolites are crystalline aluminosilicates of group IA and group IIA elements such as Na, K, Mn, Ca and are chemically represented by the empirical formula :

$$M_{2/n}O.\ Al_2O_3.\ ySiO_2.\ wH_2O$$

where y is 2 or greater, n is the cation valence, and w is the water content in the voids of the zeolite.

Structurally, zeolites are complex, crystalline inorganic polymers based on an infinitely extending framework of $AlO_4$ and $SiO_4$ tetrahedra linked to each other by sharing of oxygen ions. This framework structure contains channels or interconnected voids that are occupied by the cations and water molecules.

The structural formula of a zeolite is based on the crystal unit cell, the smallest unit of structure, represented by

$$M_{x/n} [(AlO_2)_x (SiO_2)_y]. \ wH_2O$$

where n is the valence of cation M, w is the number of water molecules per unit cell, x and y are the total number of tedrahedra per unit cell, y/x usually having values of 1-5.

Zeolites may be naturally derived or synthetically manufactured. The synthetic zeolites being preferred for use herein. Suitable zeolites for use herein include zeolite A, zeolite P, zeolite Y, zeolite X, zeolite DAY, zeolite ZSM-5, or mixtures thereof. Most preferred is zeolite A.

According to the present invention the zeolite is preferably hydrophobic. This is typically achieved by increasing the molar ratio of the $SiO_2$ to $AlO_2$ content such that the ratio of x to y is at least 1, preferably from 1 to 500, most preferably from 1 to 6.

The absorbent structure according to the present invention typically comprises from 0 to 600 gm-2, more preferably from 10 to 300 gm-2 most preferably from 40 to 80 gm-2, of zeolite based on 100% purity or a mixture thereof.

The carbon material suitable for employment herein is the material well known in the art as an absorber for organic molecules and/or air purification purposes. Carbon suitable for use herein is available from a number of commercial sources under the trade names such as CALGON Type "CPG", Type SGL, Type "CAL" and type "OL". Often such material is referred to as "activated" carbon or "activated" charcoal. Typically it is available in the form of an extremely fine, dusty particles (e.g., 0.1 to 300μm) having large surface areas (200 - several thousand $m^2$/g.) It is to be understood that any of the "air purifying" or "activated" carbons of commerce can be used in the practice of this invention. Alternatively carbon cloth or carbon paper might also be use herein.

The absorbent structure according to the present invention typically comprises from 0 to 600 gm-2, more preferably from 10 to 300 gm-2 most preferably from 40 to 200 gm-2, of carbon material or a mixture thereof.

Silica material for use herein might be naturally derived or synthetically manufactured. Silica, i.e. silicon dioxide $SiO_2$ exists in a variety of crystalline forms and amorphous modifications, any of which are suitable for use herein. In particular, silicas having a high surface area or in agglomerated form are preferred. Silica molecular sieves are not considered to be within the definition of silica as used herein. Preferably the silica is in a highly purified form such that is contains at least 90%, preferably 95%, more preferably 99% silicon dioxide. Most preferably the silica is silica gel having a 100% silica content. Alternatively, the silica may be provided from other sources such as metal silicates including sodium silicate.

Suitable silicate for use herein are for instance Silica gel 123® or Syloblanc 82® available from Grace GmbH.

The absorbent structure according to the present invention typically comprises from 0 to 600 gm-2, more preferably from 10 to 300 gm-2 most preferably from 40 to 80 gm-2, of silica based on 100% purity or a mixture thereof.

In a preferred embodiment herein the absorbent structures comprise carbon material (X), zeolite (Y) and/or silica (Z) as the odor control agents in weight percent expressed to the total weight of odour control agents, such that X, Y, and Z independently vary within the range of 0 to 100% and wherein at least X, Y or Z is not 0.

As another essential feature the absorbent structures according to the present invention have a water absorption capacity of at least 0.06 grams per $cm^2$. Typically the water absorption capacity of the structure expressed in grams per square cm of the absorbent structure is from 0.08 to 6, preferably from 0.1 to 3, more preferably from 0.2 to 1.5 and most preferably 0.25 to 0.65.

Typically, the absorbent structure according to the present invention also has a water absorption capacity of at least 2 grams per gram of absorbent structure, preferably from 3.5 to 250, more preferably from 4.5 to 125, even more preferably from 8 to 60 and most preferably from 10 to 25 grams per gram.

The water absorption capacity is determined according to the water absorption test method as described herein after.

This effective water absorption capacity is typically achieved by the presence of water absorbing means. The absorbent means may include any material commonly used in absorbent structures such as comminuted wood pulp which is generally referred to as airfelt. Examples of other suitable absorbent means include creped cellulose wadding, meltblown polymers including coform, cross-linked cellulosic fibers, tissue including wraps, absorbent foams, absorbent sponges, absorbing gelling materials and any combinations thereof. Highly preferred herein are absorbent gelling materials.

Absorbent gelling materials (sometimes referred to as 'super-sorbers') are materials which have fluid absorbing properties. Such materials form hydrogels on contact with water. Highly preferred type of hydrogel-forming, absorbent gelling material are based on polyacids, especially polyacrylic acid. Hydrogel-forming polymeric materials of this type are those which, upon contact with fluids (i.e., liquids) such as water, imbibe such fluids and thereby form hydrogels. These preferred absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. Acrylic acid grafted starch materials are of this latter type. Thus, the preferred absorbent gelling materials include hydrolyzed acrylonitrile grafted starch, acrylic acid grafted starch, polyacrylates, maleic anhydride-based copolymers and combinations thereof. Especially preferred absorbent gelling materials are the polyacrylates and acrylic acid grafted starch.

Whatever the nature of the polymer components of the preferred absorbent gelling materials, such materials will in general be slightly cross-linked. Crosslinking serves to render these preferred hydrogel-forming absorbent materials substantially water-insoluble, and cross-linking also in part determines the gel volume and extractable polymer characteristics of the hydrogels formed therefrom. Suitable cross-linking agents are well known in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer materials; and (4) polyvalent metal compounds which can from ionic cross-linkages. Cross-linking agents of the foregoing types are described in greater detail in Masuda et al; U.S. Patent 4,076,663; Issued February 28, 1978. Preferred cross-linking agents are the di- or polyesters of unsaturated mono-or polycarboxylic acids with polyols, the bisacrylamides and the di-or triallyl amines. Especially preferred cross-linking agents are N,N'-methylenebisacrylamide, trimethylol propane triacrylate and triallyl amine. The cross-linking agent will generally comprise from about 0.001 mole percent to 5 mole percent of the preferred materials. More preferably, the cross-linking agent will comprise from about 0.01 mole percent to 3 mole percent of the gelling materials used herein.

The preferred, slightly cross-linked, hydrogel-forming absorbent gelling materials will generally be employed in their partially neutralized form. For purposes described herein, such materials are considered partially neutralized when at least 25 mole percent, and preferably at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to as the "degree of neutralization". Typically, commercial absorbent gelling materials have a degree of neutralization somewhat from 25% to 90%.

The preferred absorbent gelling materials used herein are those which have a relatively high capacity for imbibing fluids encountered in the absorbent structures; this capacity can be quantified by referencing the "gel volume" of said absorbent gelling materials.

Gel volume is typically defined by taking as a reference liquid, synthetic urine. Gel volume can be defined in terms of the amount of synthetic urine absorbed by any given absorbent gelling agent buffer and is specified as grams of synthetic urine per gram of gelling agent. Gel volume in synthetic urine (see Brandt, et al, below) can be determined by forming a suspension of about 0.1-0.2 parts of dried absorbent gelling material to be tested with about 20 parts of synthetic urine. This suspension is maintained at ambient temperature under gentle stirring for about 1 hour so that swelling equilibrium is attained. The gel volume (grams of synthetic urine per gram of absorbent gelling material) is then calculated from the weight fraction of the gelling agent in the suspension and the ratio of the liquid volume excluded from the formed hydrogel to the total volume of the suspension. The preferred absorbent gelling materials useful in this invention will have a gel volume of from about 20 to 70 grams, more preferably from about 30 to 60 grams, of synthetic urine per gram of absorbent gelling material.

Another feature of the most highly preferred absorbent gelling materials relates to the level of extractable polymer material present in said materials. Extractable polymer levels can be determined by contacting a sample of preferred absorbent gelling material with a synthetic urine solution for the substantial period of time (e.g., at least 16 hours) which is needed to reach extraction equilibrium, by then filtering the formed hydrogel from the supernatant liquid, and finally by then determining the polymer content of the filtrate. The particular procedure used to determine extractable polymer content of the preferred absorbent gelling agent buffers herein is set forth in Brandt, Goldman and Inglin; U.S. Patent 4,654,039; Issues March 31,1987, Reissue 32,649, The absorbent gelling materials which are especially useful in the absorbent structures herein are those which have an equilibrium extractable content in synthetic urine of no more than about 17%, preferably no more than about 10% by weight of the absorbent gelling material.

The absorbent gelling materials herein before described are typically used in the form of discrete particles. Such absorbent gelling materials can be of any desired shape, e.g., spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used.

The size of the absorbent gelling material particles may vary over a wide range. For reason of industrial hygiene, average particle sizes smaller than about 30 microns are less desirable. Particles having a smallest dimension larger than about 2mm may also cause a feeling of grittyness in the absorbent structure, which is undesirable from a consumer aesthetics standpoint. Furthermore, rate of fluid absorption can be affected by particle size. Larger particles have very much reduced rates of absorption. Preferred for use herein are absorbent gelling material particles substantially all of which have a particle size of from about 30 microns to about 2mm. "Particle Size" as used herein means the weighted average of the smallest dimension of the individual particles.

Suitable absorbent gelling material for use herein are for instance Drytech XZ91002.01® available from Dow Chemical, Favor SXM 300® available from Chemische Fabrik Stockhausen, Aqualic L-76® available from Nippon Shokubai or Aridall ASAP 2000® available from Chemdal. Preferred for use herein is a cross-linked sodium polyacrylate XZ 9589001® available from Dow Chemicals or L74® available from Shokubay.

The amount of absorbent gelling material particles used in the absorbent structures according to the present invention will typically range from 0 $gm^{-2}$ to 600 $gm^{-2}$, preferably from 10 $gm^{-2}$ to 100 $gm^{-2}$, more preferably from 20 $gm^{-2}$ to 70 $gm^{-2}$, and most preferably from 25 $gm^{-2}$ to 50 $gm^{-2}$.

Advantageously by using absorbent gelling materials it is possible to provide absorbent structures for containing odor and liquid in inanimate places, which contain less hydrophilic fibers for a given absorption capacity and which advantageously have small thickness. The caliper of the absorbent structure according of the present invention is typically from 0.4 to 3 mm, preferably from 0.8 to 2 and more preferably from 1 to 1.5 mm. Advantageously the thin layered absorbent structures according to the present invention are easily pliable and thus especially suitable to be used as a cleaning wipes for cleaning hard surfaces too.

In the preferred embodiment the absorbent structure comprises a first and a second layer of fibrous material and an intermediate layer between the first and second layer of fibrous material. The intermediate layer typically comprises the odour control means, typically the odour control agent, and the water absorbing means, typically the absorbent gelling material. The combination of an odour control means with an absorbent gelling material in the layered absorbent structures according to the present invention results in both optimum odour and liquid control on inanimate places contaminated with odour and spillage. Indeed without to be bound by any theory, the presence of absorbent gelling material boosts the odour control capacity of the odour control agent and the presence of odour absorbing agents like carbon material, zeolite material and/or silicate material further helps the liquid absorption and retention capacity of the absorbent gelling material.

The absorbent structures according to the present invention may comprise additional layers besides the first and the second layer of fibrous material. It may comprise a topsheet overlaying the first layer of fibrous material on the side facing away from the intermediate layer. It may comprise a backsheet overlaying the second layer of fibrous material on the side facing away from the intermediate layer. The topsheet and backsheet may respectively be a single layer of multiple layers of the same or different materials. The material might be chosen depending on the function/ benefit to deliver taken also in consideration other considerations like cost, process feasibility and the like.

Typically suitable topsheet for use herein, as a whole and hence each layer individually, needs to be compliant, soft feeling, and non-irritating to the skin. It also can have elastic characteristics allowing it to be stretched in one or two directions. The topsheet may be formed from any of the materials available for this purpose and known in the art, such as woven and non woven fabrics and films such as liquid permeable apertured polymeric film. The apertured topsheet might be chosen to facilitate liquid transport from the outer facing surface towards the intermediate layer. Apertured topsheet further participates to the odor control benefit.

Depending on the end benefit desired the backsheet might be made of any material, as for the topsheet. In one embodiment the backsheet is preferably impervious to liquids. It can thus be manufactured from a thin plastic film, although other flexible liquid impervious materials can also be used. As used herein, the term "flexible" refers to materials that are compliant and will readily be pliable. The backsheet also can have elastic characteristics allowing it to stretch in one or more directions. Typically the backsheet can comprise a woven or nonwoven material, polymeric films such as thermoplastic films of polyethylene or polypropylene, or composite materials such as a film-coated nonwoven material. Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385.

In a preferred embodiment both the topsheet and the backsheet can permit air and/or water vapors to enter and escape from the absorbent structures, i.e., be breathable, while still preventing liquid from passing through the backsheet. Breathable topsheets and/or backsheets might comprise several layers, e.g. film plus non-woven structures, can be used. Breathable absorbent structures are preferred herein as they contribute to further improve the control of odor. Furthermore the use of the breathable topsheet and/or breathable backsheet further contributes to a clean and dry facing outer surface (topsheet and/or backsheet), such that the surface feels dry to the touch.

Typically the absorbent structures according to the present invention have an air permeability (as measured by the air permeability test method described herein after) higher than 100 l/m$^2$/s, preferably higher than 300 l/m$^2$/s, and more preferably higher than 600 l/m$^2$/s.

Typically the absorbent structures according to the present invention have a water vapour permeability (as measured by the water vapour permeability test method described herein after) higher than 300 (g) / (m$^2$/24hrs) preferably higher than 600 (g) / (m$^2$/24 hrs), and more preferably higher than 900 (g) / (m$^2$/ 24 hrs).

The absorbent structures according to the present invention may comprise a means to provide a 'scent signal' in the form of a pleasant odor which signals the removal of odor during use of the absorbent structures. Any technology known to those skilled in the art and compatible with the needs of the present absorbent structures might be used herein. Suitable means for use herein include moisture-activated encapsulated perfumes. Moisture-activated encapsulated perfumes include any encapsulated perfume system which will release the perfume when wetted by liquid typically water. Preferably, moisture-activated perfumes include cyclodextrin/perfume inclusion complexes, polysaccharides matrix perfume microcapsules and mixtures thereof. Cyclodextrin/perfume inclusion complexes are very stable in dry state. Even the very volatile perfume molecules are bound in the cavity of the cyclodextrin molecules and do not provide perceptible odor. Upon wetting by a liquid like aqueous fluid, the perfume is released to provide a burst of fragrance. A great variety of perfumes can be used to accommodate a variety of consumer preferences. In polysaccharide matrix perfume microcapsules, the perfume is dispersed as minute droplets in , e.g., a starch/dextrin solid cellular matrix. Moisture swells and softens the polysaccharide matrix to release the encapsulated perfume. Preferably cyclodextrin/perfume inclusion complexes and the polysaccharide matrix perfume microcapsules contain volatile perfume.

In the absorbent structures according to the present invention the absorbent gelling material and odor control agent may be incorporated as a powder, a granulate, a particulate. When used in a granulate or particulate form the absorbent gelling material as well as the odor control agent may be granulated separately and then mixed together or granulated together. The odor control agents and the absorbent gelling materials may be incorporated into the absorbent structure typically between the two layers of fibrous materials by any conventional method known to those skilled in the art. In a preferred execution herein the two fibrous layers typically extend beyond the intermediate layer forming edge portions. The first and the second fibrous layer are joined along their edge portions by any conventional attachment method known to those skilled in the art, leaving the odor control agents and absorbent gelling materials free to slide between the surfaces of the two fibrous layers between which they are included. The two fibrous layers might be joined at their edge portions by for example sewing, gluing, pressing, melting, wave bonded (i.e., ultrasonic, IR, UV). Typically a continuous line of adhesive is preferred. This ensures that the edge portions of the layered structure stick and any loose odor control agent and absorbent gelling material present in the intermediate layer do not fall out of the layered absorbent structure. The preferred absorbent structures according to the present invention further comprise additional bonding areas (the so called bonding areas) where the two fibrous layers are directly bonded together in absence of the intermediate layer (this on top of the attachment at the external edge portions of the two coinciding fibrous layers). These bonding areas (or lines), separate adjacent areas formed by the intermediate layer, the so called discontinuous intermediate layer. These bonding might be made by using any conventional method including the use of thermoplastic bonding material, wax, crosslinking agent or conventional glue. Preferably these bonding areas are made by continuous lines of adhesive or thermobonding.

These bonding areas have the advantage to help the sizability and integrity of the absorbent structure (avoiding leakage of actives as well as active concentration to limited area of the structure, especially in non-horizontal usage conditions). These bonding areas contribute to the overall effectiveness of the absorbent structure in any usage conditions included non-horizontal conditions. By sizability it is meant herein that it is possible for the user to cut along the bonding areas, thereby obtaining absorbent structures of any desired size. This allows cutability of the structure for any desirable size and accordingly various applications. Any method known to those skilled in the art might be used herein to bond the first and second fibrous layer together in absence of the intermediate layer at the so called bonding areas and allow easy cutability without impairing the integrity of the structure. Accordingly a suitable way is for example to provide bonding areas/lines of enough width so that by cutting along a line along for example the middle of such areas/lines the function of such bonding lines/areas towards the areas formed by the discontinuous intermediate layer is maintained. Another way is by providing bonding areas with several parallel independent bonding lines allowing for the possibility to cut between two parallel bonding lines. A pre-perforated system might also be provided. Any configuration of the bonding areas is suitable herein so as to provide desirable sizability.

In a preferred execution herein the two layers of fibrous material are further joined together by particles of thermoplastic polymeric organic material distributed and mixed with the odor control agents and absorbent gelling materials, i.e., the two fibrous layers might be bonded together in area having the intermediate layer between them too. Actually the actives might be incorporated in the structure in accordance with the disclosure of WO 94/01069. The quantity of thermoplastic polymeric organic materials (preferably particles of polyethylene) distributed and mixed with the odor control agents and absorbent gelling materials is between 5g/m$^2$ and 150g/m$^2$, preferably from 10 to 60 g/m$^2$. The thermoplastic polymeric organic materials suitable for use herein are those having a melt flow index (M.F.I.) evaluated by the ASTM method D 1238-85 under conditions 190/2.16, of at least 25g/10 min., preferably at least 40g/10 min., and even preferably at least 60g/10 min. If the fibrous layers are made of an air-laid short cellulose fibre material, it is preferred to use a thermoplastic polymeric organic material composed of particles of high-density polyethylene with maximum dimensions of 400 microns, characterized by a melt flow index of 50g/10min., of which the quantity distributed is between 5g/m2 and 15g/m2. In the preferred embodiment of the invention the thermoplastic material is referred to as being in the form of particles. It is to be understood that these 'particles' need not necessarily be in the form of generally spherical bodies. They could for example be in the form of fibrils. These are very short fibres and suitable fibrils include those available from Lextar V-O-F of Rotterdam, Netherlands, under the trade mark PULPEX PE, these are polyethylene fibres having an average length of from 0.6 to 1.2 mm.

During the manufacturing process the mixture is heated such that the thermoplastic polymeric organic material (preferably polyethylene) melts and glues the first and second layer of fibrous material together. The bond between the first and second fibrous layer is formed at discrete spaced-apart points generated by the melting of the thermoplastic polymeric material. In practice the odor control agent and absorbent gelling material are affected only to a very marginal extent by the melting of the thermoplastic polymeric material and thus remain trapped between the two fibrous layers without being substantially bonded thereto.

Alternatively, the polyethylene powder may be replaced by any conventional wax or crosslinking agent, or conventional glue for instance those commercially available from ATO Findley under the name H20-31® to glue the fibrous layers of the absorbent structure together. Advantageously this method step allows to avoid the heating step necessary when using particle of thermoplastic polymeric material like particles of polyethylene.

In one execution the intermediate layer as called herein between the two fibrous layers (first and second layer) may further comprise one or more additional layers. Such layers might be made of any material and be air and/or water vapor permeable and/or liquid permeable or liquid impermeable depending on the desired end result to achieve. Typically such additional layer might constitute a physical separation between the absorbing gelling material and the odor control agents, or even a physical separation between different kinds of odor control agents as desired.

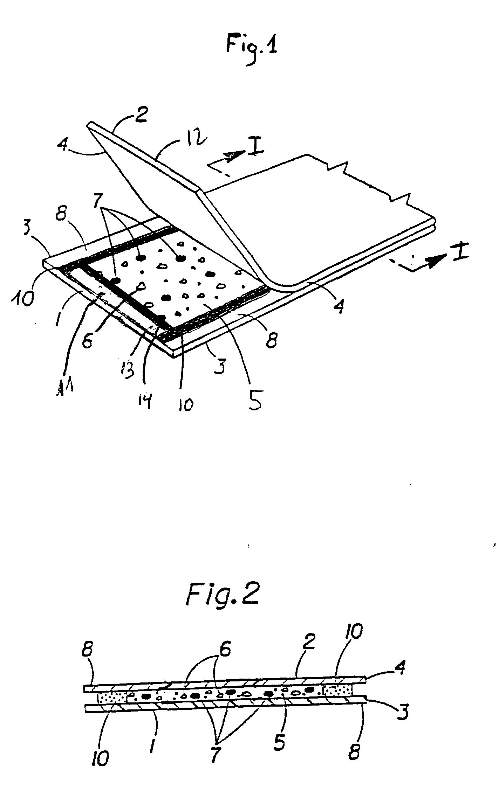

Figures 1 and 2 show an absorbent structure suitable for use in the method of containment of odor and liquid according to the present invention. In Figure 2 one of the layers is partially raised to show its construction more clearly.

In Figure 1 it is possible to distinguish a first fibrous layer 2 and a second fibrous layer 1 in the form of two continuous strips of the same width and length, which are superposed so that their respective longitudinal edges 3 and 4 coincide and so that their respective lateral edges 12 and 13 coincide. The first and second layer of fibrous material can be made of the same or different materials. Between the two fibrous layers 1 and 2 there is an intermediate layer 5 made of mixture of particles of odor control agent 6 and absorbent gelling material 7, the width and length of the intermediate layer 5 is less than the width and length of the two outer fibrous layers 1 and 2 which extend beyond the intermediate layer 5 laterally forming two longitudinal edge portions 8 at their respective longitudinal edges 3 and 4 and lateral edge portions 11 at their respective lateral edges 12 and 13. Preferably the intermediate layer also comprises particles of a thermoplastic polymeric organic material (not shown). Two lines of adhesive 10 are applied to the two sides of the intermediate layer 5 on the longitudinal edge regions 8 of the two outer fibrous layers 1 and 2 so as to eliminate possibility of particles of odor control agent and absorbent gelling material escaping from the longitudinal edges of the layered structure, which correspond to the superposed edges 3 and 4 of the two fibrous layers. Typically two lines of adhesive 14 are applied to the side of the lateral edge portions 11 of the two outer fibrous layers 1 and 2 so as to eliminate possibility of particles of odor control agent and absorbent gelling material escaping from the lateral , edges of the layered structure, which correspond to the superposed edges 12 and 13 of the two fibrous layers. Alternatively the lateral and longitudinal edges portions might be joined/sealed by other conventional means.

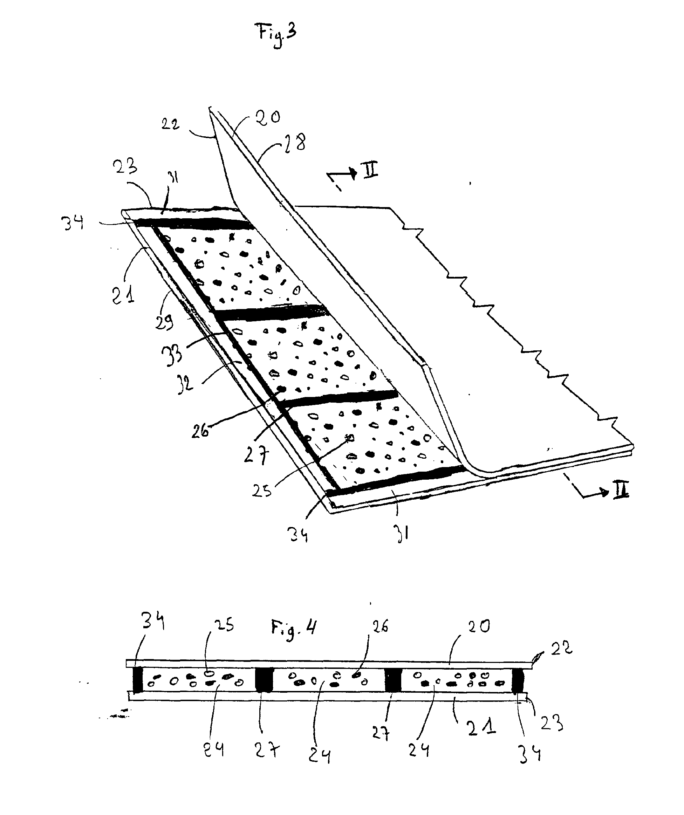

Figures 3 to 6 show an absorbent structure as claimed in the present invention. In Figures 3 and 5 one of the layers is partially raised to show its construction more clearly.

In Figures 3 to 6 it is possible to distinguish a first fibrous layer 20 and a second fibrous layer 21 in the form of two continuous strips of the same width and length, which are superposed so that their respective longitudinal edges 22 and 23 coincide and so that their respective lateral edges 28 and 29 coincide. Between the two fibrous layers 20 and 21 there are areas formed by the discontinuous intermediate layer 24 made of mixture of particles of odor control agent 25 and absorbent gelling material 26. The discontinuous intermediate layer may preferably comprise particles of a thermoplastic polymeric organic material (not shown) besides the odor control agent 25 and absorbing gelling material 26. The two fibrous layers 20 and 21 have a width and length which is more than the width and length the discontinuous intermediate layer 24.

By 'discontinuous intermediate layer' it is to be understood herein that the intermediate layer is not continuous as opposed to the first and second layer of fibrous material which both are continuous. More particularly by discontinuous intermediate layer is to be understood that the intermediate layer in its whole is constituted by areas of intermediate layer separated by adjacent bonding areas 27 where the first and the second layer of fibrous material are bonded together in absence of any intermediate layer in between the fibrous layers at the bonding areas. Any configuration, size of the areas forming the discontinuous intermediate layer may be suitable to be used herein. Typically the areas formed by the discontinuous intermediate layer in respect to the bonding areas when considering the total surface area of the absorbent structure according to the present invention represents at least 50%, preferably from 70% to 99% and more preferably from 75% to 95%.

In the preferred execution as illustrated in Figures 3 to 6, two lines of adhesive 34 are applied on the longitudinal edge regions 31 of the two outer fibrous layers so as to eliminate possibility of particles of odor control agent and absorbent gelling material escaping from the longitudinal edges of the layered structure. Two lines of adhesive (33 in Figure 3) are applied on the lateral edge regions 32 of the two outer fibrous layers so as to eliminate possibility of particles of odor control agent and absorbent gelling material escaping from the lateral edges of the layered structure. The discontinuous intermediate layer defines areas separated by bonding areas 27. Bonding areas 27 are applied to the longitudinal sides of the areas formed by the discontinuous intermediate layer 24, typically in the form of continuous lines of thermobonding material or conventional adhesive, thereby delimiting these areas and eliminating the possibility that particles of odor control agent and absorbent gelling material escape from those areas. It is to be understood herein that these bonding areas 27 may have any configuration, straight or curved thereby delimiting areas formed by the discontinuous intermediate layer having any form or size.

These bonding areas 27 have the advantage to help the sizability and integrity of the absorbent structure.

Figures 5 and 6 differ from Figures 3 and 4 in that the absorbent structures illustrated have an additional layer 30 (so called backsheet) in the form of a continuous strip of the same wide and length as the second layer of fibrous material 21 and overlaying the second layer of fibrous material 21 on the side facing away from the discontinuous intermediate layer 24. The layer 30 is joined to the layer 21 by any conventional means, e.g., by gluing them together. This backsheet layer is preferably liquid impervious and/or air and/or water vapor permeable.

Any process known to those skilled in the art might be suitable to provide the absorbent structures according to the present invention. Typically the herein so called second fibrous layer is provided, then the intermediate layer comprising the water absorbing means and odor control means is applied thereto as desired (continuously (cf. Figures 1 and 2) or discontinuously (Figures 3 to 6)). In the preferred embodiment herein wherein the intermediate layer comprises thermoplastic polymeric material, the structure is heated to melt the particle of polymeric material for example by means of a radiant heating element. Then typically adhesive lines are applied to this layer on the lateral and longitudinal edges portions and optionally (see Figures 3 to 6) at areas called herein bonding areas, the first fibrous layer is then superposed on the second fibrous layer. The structure is bonded by for example subjecting it to moderate pressure.

These structures according to the present invention might be delivered as such in a box comprising superposed sheet-like absorbent structures of any desired size, typically sheets of rectangular shape with several compartments made of the discontinuous intermediate layer, or by other conventional dispensing devices like roll dispensing devices.

Naturally the details of the construction may be varied widely from those described and illustrated without thereby departing from the scope of the present invention. In particular, there may be more than one intermediate layer and fibrous layer enclosing it, thus forming several pairs of fibrous layers each enclosing an intermediate layer.

Absorbent structure tests

To determine the water absorption capacity of the absorbent structures according to the present invention the DIN absorptive capacity test is used.

1) Measuring principle

The DIN - absorptive capacity is determined on samples with a surface of 100 square centimeters after a 300-second-period of soaking the sample and a 120-second-period of dripping.

2) Instruments and testing aids

- sample cutter (e.g.: NAEF sample cutter M53 with cutting die 100cm$^2$)
- scales (e=0,001g)
- water box (length x width x height = 150 x 80 x 130 mm) or aquarium
- watch-glass
- tweezers
- stopwatch
- distilled water
- clamp in order to fix the sample: ca. 11 cm wide

The clamp has to be installed on the horizontal bare (also called crossbeam) in an angle of 5° to the vertical. The horizontal bare must be freely movable towards the bottom section and able to lock in the top position.

3) Proceeding

The absorbent structure to be tested is cut into samples of 100 square cm.
The climatized, cut and dry sample is weighed with a precision of 0,001 g (reported as $m_k$).
Afterwards the sample is clamped by means of a clamp on a horizontal bare positioned above the water box and lowered into the distilled water until it's completely immersed. During the period of soaking the sample should be under the surface of the water and it must be guaranteed that water can reach the sample from all sides.
After a period of 300±3 secs the sample is taken out of the water by rising the horizontal bare and after this the horizontal bare is locked in the top position, i.e., the sample is completely out of the water of the water box. After a dripping period of 120±3 secs the sample is transferred onto a watch-glass in order to determine the weight $m_n$ (by opening the clamp the sample falls onto the watch-glass underneath) and is weighted with a precision of 0,01 g. (Tara must be set to zero before)

4) Evaluation

$$\text{DIN-Absorptive Capacity (g/100 cm}^2) = m_n - mk$$

$m_n$ = weight of wet sample in g
$m_k$ = weight of dry sample in g

The water absorption capacity can be reported in grams per square cm of the absorbent structure tested as well as in grams per gram of absorbent structure tested.

Air & Vapor Permeability Test on absorbent structure according to the present invention

The Vapor permeability test is utilized to quantify the vapor transmission properties of breathable absorbent structures.

Basic Principle of the Methods:

The basic principle of the test is to quantify the extent of water vapor transmission of an absorbent structure. The test method that is applied is a standard one, namely ASTM E 96 - 80, Procedure B - Water Method at 23°C. The test is performed in a stable temperature/humidity laboratory maintained at a temperature of 23° C at 50% RH for a period of 24 hours.
The vapor permeability value is determined as the weight loss divided by the open area of the sample holder and quoted per day.

$$\text{i.e. Vapor Permeability} = \text{Weight Loss (g)} / (\text{m}^2/ 24 \text{ hrs})$$

Air Permeability test:

The air permeability test is utilized to assess the ability of an absorbent structure to circulation/exchange air.

Basic Principle of the Methods:

The basic principle of the test is to evaluate the resistance of an absorbent structure to the passage of air. In this test, the volume (or amount) of air that flows through an absorbent structure of given dimensions under standard conditions (of 23 °C /50% RH) is measured. The instrument utilized for the test is: Air Permeabilimeter FX 3300 manufactured by TexTest AG Switzerland.
Samples should be allowed to equilibrate in the test environment for at least 4 hrs prior to commencement of the measurement. The absorbent structure (having dimensions exceeding 5 cm$^2$ the dimensions of the measurement head) is placed on the device as instructed by the manufacturer. An aspiration pump set to generate a pressure of 1210 kPa that sucks air through the sample structure. The device measures the volume of air flow and the pressure drop across the orifices that contains the sample and measurement head. Finally the device generates a value of air permeability in the units of liters/m2/s.

Caliper Measurement

The Average caliper of the absorbent structure is determined. For products that are inherently flat the caliper at representative points (at least 5) of the product is measured to determine an average value.

The present invention is illustrated by following examples

Absorbent structures as exemplified in Figures 1, 3 and 5 have been made.

These absorbent structures (cf Figures 1 and 3) comprise:

- a first fibrous layer formed from thermal bonded air laid web of cellulose fibers 70g/m2,
- a continuous intermediate layer (Figure 1) or discontinuous intermediate layer (Figures 3 and 5) formed from a mixture of 25g/sqm of absorbent gelling material commercially available from Shokubay under name L74®, of 52g/sqm of zeolite commercially available from Degussa under name Wessalith CS®, of 72g/sqm of carbon commercially available from Chemviron SC and 20g/sqm of particles of polyethylene powder (Verplast verlene D2000®)
- a second fibrous layer formed from thermal bonded air laid web of cellulose fibers 70g/m2.

The absorbent structure of Figure 5 differs from previously exemplified ones in that a laminate of air laid of cellulose 70g/m2 with a plastic film backsheet 10g/m2 (polyethylene film Exton) is used as the second fibrous layer and backsheet.

**Claims**

1. A method of containment of odor and liquid in an inanimate place by disposing in said place a disposable absorbent structure in the form of a sheet, said absorbent structure comprising an odor control means and having a water absorption capacity of at least 0.06 grams per square cm

2. A method according to claim 1, wherein said absorbent structure is a layered absorbent structure comprising at least one layer of fibrous material as substrate for the odor control means.

3. A method according to any of the preceding claims, wherein said absorbent structure is a layered absorbent structure comprising a first and a second layer of fibrous material and an intermediate layer comprising said odor control means

4. A method according to any of the preceding claims, wherein the water absorption capacity of the absorbent structure is from 0.08 to 6 grams per square cm and wherein the water absorption capacity is typically of at least 2 grams per gram of the absorbent structure, preferably of from 3.5 to 250 grams per gram.

5. A method according to any of the preceding claims 3 or 4, wherein the intermediate layer comprises a water absorbing means together with said odor control means.

6. A method according to any of the preceding claims wherein the inanimate place is a refrigerator, kitchen, bathroom, toilet, cupboard, wardrobe, waste bins, ashtray, picnic and lunch basket, sport and travel bag, laundry bag, shoes.

7. A layered absorbent structure, particularly suitable for neutralizing odor and absorbing liquid in inanimate places, comprising a first and second layer of fibrous material and a discontinuous intermediate layer between said first and second layer of fibrous material, the discontinuous intermediate layer comprising carbon material and a water absorbing means, the discontinuous intermediate layer defining areas which are separated by bonding areas where the first and second layer of fibrous material are bonded together.

8. A layered absorbent structure, particularly suitable for neutralizing odor and absorbing liquid in inanimate places, comprising a first and a second layer of fibrous material and a discontinuous intermediate layer between said first and second layer of fibrous material, the discontinuous intermediate layer comprising an odor control means and a water absorbing means, the discontinuous intermediate layer defining areas separated by bonding areas where the first and second layer of fibrous material are bonded together, the absorbent structure further comprising a means to provide a 'scent signal' in the form of a pleasant odor which signals the removal of odor during use of the absorbent structure.

9. A layered absorbent structure, particularly suitable for neutralizing odor and absorbing liquid in inanimate places, comprising a first and a second layer of fibrous material and a discontinuous intermediate layer between said first and second layer of fibrous material, the discontinuous intermediate layer comprising an odor control means and a water absorbing means, the discontinuous intermediate layer defining areas separated by bonding areas where the first and second layer of fibrous material are bonded together, the absorbent structure further comprising attachment means.

10. An absorbent structure, particularly suitable for neutralizing odor and absorbing liquid in inanimate places, said absorbent structure being in the form of a sheet and comprising an odor control means and having a water absorption capacity of at least 0.06 grams per square cm, the absorbent structure being provided with antislipping properties.

11. An absorbent structure, particularly suitable for neutralizing odor and absorbing liquid in inanimate places, said absorbent structure being in the form of a sheet and comprising an odor control means and having a water absorption capacity of at least 0.06 grams per square cm, the absorbent structure being provided with an indicator which indicates the end of the life time of the absorbent structure.

12. A layered absorbent structure, particularly suitable for neutralizing odor and absorbing liquid in inanimate places, comprising a first and second layer of fibrous material and a discontinuous intermediate layer between said first and second layer of fibrous material, the discontinuous intermediate layer comprising an odor control means and a water absorbing means, the discontinuous intermediate layer defining areas separated by bonding areas where the first and second layer of fibrous material are bonded together, the absorbent structure further comprising a backsheet overlaying the second layer of fibrous material on the side facing away from said intermediate layer

13. A method according to claims 1 to 6 or an absorbent structure according to any of the claims 7 to 12, wherein said odor control means is an odor control agent, typically selected from the group consisting of carbonates, bicarbonates, phosphates, sulphates, carboxylic acids, carbon materials, baking soda, natural or synthetic clays, kieselguhr, silicates, zeolites, diatomaceous earth, bentonite, starch, mucopolysaccharides, cyclodextrine and derivatives thereof, chelating agents, ion exchange resins, perfumes, pH buffering means, antimicrobial agents and a mixture thereof and preferably is carbon material and/or zeolite material and/or silicate material.

14. A method or absorbent structure according to claim 13, wherein the level of the odor control agent or a mixture thereof in the absorbent structure is from 10 gm-2 to 600 gm-2, preferably from 50 to 300 gm-2, more preferably from 100 gm-2 to 200 gm-2 and most preferably from 120 gm-2 to 150gm-2.

15. An absorbent structure according to any of the preceding claims 10 or 11, wherein the absorbent structure comprises a water absorbing means together with said odor control means and wherein preferably the absorbent structure is a layered absorbent structure comprising a first and a second layer of fibrous material and an intermediate layer between said first and second layer, said intermediate layer preferably comprising the water absorbing means together with the odor control means.

16. A method according to claim 5 or an absorbent structure according to any of the preceding claims 7 to 9 or 12 to 15, wherein said water absorbing means is an absorbent gelling material.

17. A method or absorbent structure according to claim 16, wherein the absorbent gelling material is hydrolyzed acrylonitrile grafted starch, acrylic acid grafted starch, polyacrylate, maleic anhydride-based copolymer and combinations thereof and more preferably is a polyacrylate and/or acrylic acid grafted starch.

18. A method or absorbent structure according to any of the preceding claims 16 or 17, wherein the absorbent structure comprises from 0.5 gm$^{-2}$ to 600 gm$^{-2}$, preferably from 10 to 100 gm$^{-2}$, and more preferably from 20gm$^{-2}$ to 70 gm$^{-2}$ of the absorbent gelling material or a mixture thereof .

19. A method according to any of the claims 3 to 6 or structure according to any of the preceding claims 7 to 9 or 12 to 18, wherein the intermediate layer of the absorbent structure further comprises thermoplastic material typically polymeric organic material and more preferably particles of polyethylene.

20. An absorbent structure according to any of the claims 12 to 14 or 16 to 19, wherein the backsheet is impervious to liquid and air and/or water vapor permeable.

21. An absorbent structure according to any of the preceding claims 7 to 9 or 12 , wherein the first and the second layer of fibrous material extend beyond the discontinuous intermediate layer to form edges portions, the first and the second layer of fibrous material being bonded to each others along each of the edge portions as well as at the bonding areas preferably by continuous lines of adhesive.

22. A method according to any of the preceding claims 2 to 6 or absorbent structure according to any of the claims 7 to 9 or 12 or 15, wherein the layer of fibrous material has/have a basis weight of between $20g/m^2$ to $150g/m^2$.

23. The use of an absorbent structure in the form of a sheet, the absorbent structure comprising an odor control means and having a water absorption capacity of at least 0.06 grams per square cm, as a refrigerator refreshing product

24. The use of an absorbent structure in the form of a sheet, the absorbent structure comprising an odor control means and having a water absorption capacity of at least 0.06 grams per square cm, as a hard surface protection sheet.

Fig.1

Fig.2

Fig.3

Fig. 4

Fig. 5

Fig. 6

## European Patent Office

## EUROPEAN SEARCH REPORT

**Application Number**

EP 00 11 0884

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 839 608 A (GILLBERG-LAFORCE GUNILLA ELSA) 24 November 1998 (1998-11-24)<br>* abstract *<br>* column 1 - column 5 *<br>--- | 1,6,13, 14,19 | A61F2/00<br>A61L9/01<br>A61L15/46<br>A23L3/3427<br>B65D81/26 |
| Y | WO 91 12031 A (PROCTER & GAMBLE) 22 August 1991 (1991-08-22)<br>* abstract *<br>* page 1, line 15-23 *<br>* page 4, line 17 - page 5, line 37 *<br>* page 14, line 17 - page 15, line 5 *<br>--- | 1-18, 20-23 | |
| Y | EP 0 045 514 A (ROTH ERICH DIPL CHEM) 10 February 1982 (1982-02-10)<br>* abstract *<br>* page 2, paragraph 2 *<br>* page 3, paragraphs 2-6 *<br>--- | 1-18, 20-23 | |
| Y | EP 0 811 390 A (PROCTER & GAMBLE) 10 December 1997 (1997-12-10)<br><br>* abstract *<br>* page 2, line 54 - page 3, line 51 *<br>* page 4, line 37-51 *<br>--- | 1-11, 13-18, 20-24 | TECHNICAL FIELDS SEARCHED (Int.Cl.7)<br><br>A61L<br>B65F<br>A23L<br>B65D |
| Y | US 5 492 675 A (BRIZARD CYRIL J C) 20 February 1996 (1996-02-20)<br><br>* abstract *<br>* column 1 - column 2 *<br>--- | 1-11, 13-18, 20-24 | |

-/--

The present search report has been drawn up for all claims

| Place of search | Date of completion of the search | Examiner |
|---|---|---|
| THE HAGUE | 24 October 2000 | Böhm, I |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**European Patent Office**

## EUROPEAN SEARCH REPORT

Application Number

EP 00 11 0884

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | US 5 733 272 A (TRINH TOAN ET AL) 31 March 1998 (1998-03-31)<br><br>* abstract *<br>* column 2, line 14 - column 3, line 12 *<br>* column 7, line 30-65 *<br>* column 9, line 7-41 *<br>* column 10, line 6-21 *<br>--- | 1-6,8, 13-18, 20-24 | |
| Y | US 5 468 447 A (BERMAS EDWARD M) 21 November 1995 (1995-11-21)<br><br>* abstract *<br>* column 1, line 8-33.65-67 *<br>--- | 1-6,8, 13-18, 20-24 | |
| A | DE 22 54 542 A (HORN GEB STOLL ANNEGRET) 9 May 1974 (1974-05-09)<br><br>* page 1, paragraphs 2,5 *<br>* page 2, paragraph 1 *<br>--- | 1,4,6, 12-14, 20,24 | |
| A | US 4 959 207 A (UEDA TSUNEHISA ET AL) 25 September 1990 (1990-09-25)<br>* abstract *<br>* column 1, line 6-25 *<br>* column 5, line 26-64 *<br>* column 6, line 28-34 *<br>--- | 1,10, 13-19 | **TECHNICAL FIELDS SEARCHED** (Int.Cl.7) |
| A | WO 98 27261 A (MOWRY LESLIE ANDERSON ;TORDIL HELEN BERNARDO (US); LIU ZAIYOU (US)) 25 June 1998 (1998-06-25)<br>* abstract *<br>* page 3, paragraphs 2-4 *<br>* page 4, paragraph 2 *<br>--- | 1,4,5, 10, 13-16,24 | |

-/--

The present search report has been drawn up for all claims

| Place of search | Date of completion of the search | Examiner |
|---|---|---|
| THE HAGUE | 24 October 2000 | Böhm, I |

EPO FORM 1503 03.82 (P04C01)

**European Patent Office**

## EUROPEAN SEARCH REPORT

Application Number

EP 00 11 0884

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| E,D | WO 00 29311 A (CHIAO I CHUN JENNIFER ;KASTURI CHANDRIKA (US); AQUINO MELISSA DEE) 25 May 2000 (2000-05-25)<br><br>* abstract *<br>* page 2 *<br>* page 4 - page 7 * | 1,4-6, 10-14, 16,18, 20,24 | |
| | | | **TECHNICAL FIELDS SEARCHED** (Int.Cl.7) |

The present search report has been drawn up for all claims

| Place of search | Date of completion of the search | Examiner |
|---|---|---|
| THE HAGUE | 24 October 2000 | Böhm, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 00 11 0884

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2000

| Patent document cited in search report | | Publication date | Patent family member(s) | | Publication date |
|---|---|---|---|---|---|
| US 5839608 | A | 24-11-1998 | US<br>CA | 5741564 A<br>2179763 A | 21-04-1998<br>23-12-1996 |
| WO 9112031 | A | 22-08-1991 | AU<br>CN<br>FI<br>PT | 7259791 A<br>1054903 A<br>923595 A<br>96733 A | 03-09-1991<br>02-10-1991<br>11-08-1992<br>29-11-1991 |
| EP 0045514 | A | 10-02-1982 | DE<br>AT<br>DE | 8021014 U<br>8454 T<br>3164876 D | 08-01-1981<br>15-08-1984<br>23-08-1984 |
| EP 0811390 | A | 10-12-1997 | AU<br>AU<br>BR<br>BR<br>CA<br>CA<br>CN<br>CN<br>EP<br>JP<br>WO<br>WO | 3147197 A<br>3287697 A<br>9709651 A<br>9709662 A<br>2257183 A<br>2257628 A<br>1225569 A<br>1225571 A<br>0811392 A<br>2000503881 T<br>9746191 A<br>9746194 A | 05-01-1998<br>05-01-1998<br>10-08-1999<br>02-05-2000<br>11-12-1997<br>11-12-1997<br>11-08-1999<br>11-08-1999<br>10-12-1997<br>04-04-2000<br>11-12-1997<br>11-12-1997 |
| US 5492675 | A | 20-02-1996 | NONE | | |
| US 5733272 | A | 31-03-1998 | AU<br>AU<br>CA<br>EP<br>JP<br>NZ<br>SG<br>WO | 693091 B<br>6447094 A<br>2157465 A<br>0691857 A<br>8508423 T<br>263688 A<br>54200 A<br>9422500 A | 25-06-1998<br>24-10-1994<br>13-10-1994<br>17-01-1996<br>10-09-1996<br>27-05-1998<br>16-11-1998<br>13-10-1994 |
| US 5468447 | A | 21-11-1995 | US<br>AU<br>WO<br>ZA | 5772959 A<br>1098495 A<br>9515187 A<br>9409478 A | 30-06-1998<br>19-06-1995<br>08-06-1995<br>11-08-1995 |
| DE 2254542 | A | 09-05-1974 | NONE | | |
| US 4959207 | A | 25-09-1990 | JP<br>JP<br>JP | 1158075 A<br>1878301 C<br>6004767 B | 21-06-1989<br>07-10-1994<br>19-01-1994 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 00 11 0884

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2000

| Patent document cited in search report | Publication date | Patent family member(s) | Publication date |
|---|---|---|---|
| US 4959207 A | | JP 1158960 A<br>JP 1921013 C<br>JP 6047003 B | 22-06-1989<br>07-04-1995<br>22-06-1994 |
| WO 9827261 A | 25-06-1998 | EP 0946808 A | 06-10-1999 |
| WO 0029311 A | 25-05-2000 | AU 1723600 A | 05-06-2000 |